# FASCICULE DE BREVET EUROPEEN

(11) **EP 1 216 667 B1**
(45) Date de publication et mention de la délivrance du brevet: **03.11.2004**
(21) Numéro de dépôt: 01403209.8
(22) Date de dépôt: 12.12.2001
(51) Int. Cl.: A61F 2/34, A61F 2/30, A61L 27/30, A61L 27/32

(54) **Cupule cotyloidienne pour prothèse de la hanche**
Gelenkpfanne für Hüftprothese
Acetabular cup for hip prosthesis

(30) Priorité: 14.12.2000 FR 0016534
(43) Date de publication de la demande: 26.06.2002
(73) Titulaire: Sulzer Orthopedie S.A., 25461 Etupes Cédex (FR)
(72) Inventeur: Bouraly, Pierre, c/o Cabinet Ballot, 25000 Besancon (FR)
(74) Mandataire: Bentz, Jean-Paul

(56) Documents cités:
- EP-A- 0 900 552
- WO-A-95/15734
- DE-A- 3 630 276
- DE-A- 19 755 536
- FR-A- 2 788 685
- FR-A- 2 793 675
- US-A- 5 571 198
- US-A- 5 782 929

## Description

La présente invention concerne une cupule cotyloïdienne pour prothèse de la hanche.

Une telle cupule est destinée à recevoir une tête sphérique d'une tige fémorale constituant une prothèse coopérant avec la cupule se logeant dans une cavité articulaire endommagée.

Préalablement à la mise en place de la tête sphérique de la tige fémorale, qui pourrait également être une tige humérale, est mis en place dans la cupule un insert en matière plastique biocompatible tel qu'un polyéthylène.

Une cupule de ce type est connue dans laquelle sont percés un certain nombre de trous filetés, initialement obstrués par des bouchons et libérés à la demande pour être traversés par des vis de fixation dans le cotyle humain avant la mise en place de l'insert interne en matière plastique.

Ce type de cupule connue présente bon nombre d'inconvénients essentiellement dus à la présence des trous destinés aux vis optionnelles qui provoquent à l'usage un fluage de l'insert en polyéthylène dans lesdits trous. De plus, la cupule cotyloïdienne généralement réalisée en métal provoque une déformation du siège de l'insert, et donc à terme, la déformation de l'articulation prothétique.

De plus, des micro particules dues au frettage de l'insert sur la cupule s'échappent par les trous ou par les interstices si ceux-ci ont reçu des bouchons filetés, car la hauteur des filetages d'où l'épaisseur de la cupule et forcément faible, afin de permettre une certaine épaisseur d'insert.

Tout cela conduit à dire que l'étanchéité est très relative dans ce type de cupule connue.

Pour remédier à ce problème d'étanchéité, il a d'abord été imaginé de revêtir la surface externe de la cupule d'hydroxy-apatite, car il est bien connu que ce matériau favorise la repousse osseuse, voir par exemple EP-A-0 900 552.

Mais, les essais ont démontré que l'étanchéité n'était pas obtenue avec ce matériau, car en fait, il s'agit d'un matériau poreux ayant un certain pouvoir d'absorption révélé par lesdits essais.

La présente invention a donc pour but de remédier à ces inconvénients et concerne à cet effet une cupule cotyloïdienne destinée à recevoir une tête sphérique d'une tige fémorale ou humérale, dans laquelle sont percés un certain nombre de trous filetés initialement obstrués par des bouchons et par des vis de fixation dans le cotyle humain avant mise en place d'un insert interne en matière plastique, caractérisée en ce qu'elle reçoit sur sa face externe un revêtement de titane poreux destiné à assurer l'étanchéité de la cupule notamment au niveau des trous non utilisés, demeurant obstrués par leurs bouchons respectifs.

La présente invention concerne également les caractéristiques qui ressortiront au cours de la description qui va suivre, et qui devront être considérées isolément ou selon toutes leurs combinaisons techniques possibles.

Cette description donnée à titre d'exemple non limitatif, fera mieux comprendre comment l'invention peut être réalisée en référence aux dessins annexés sur lesquels :

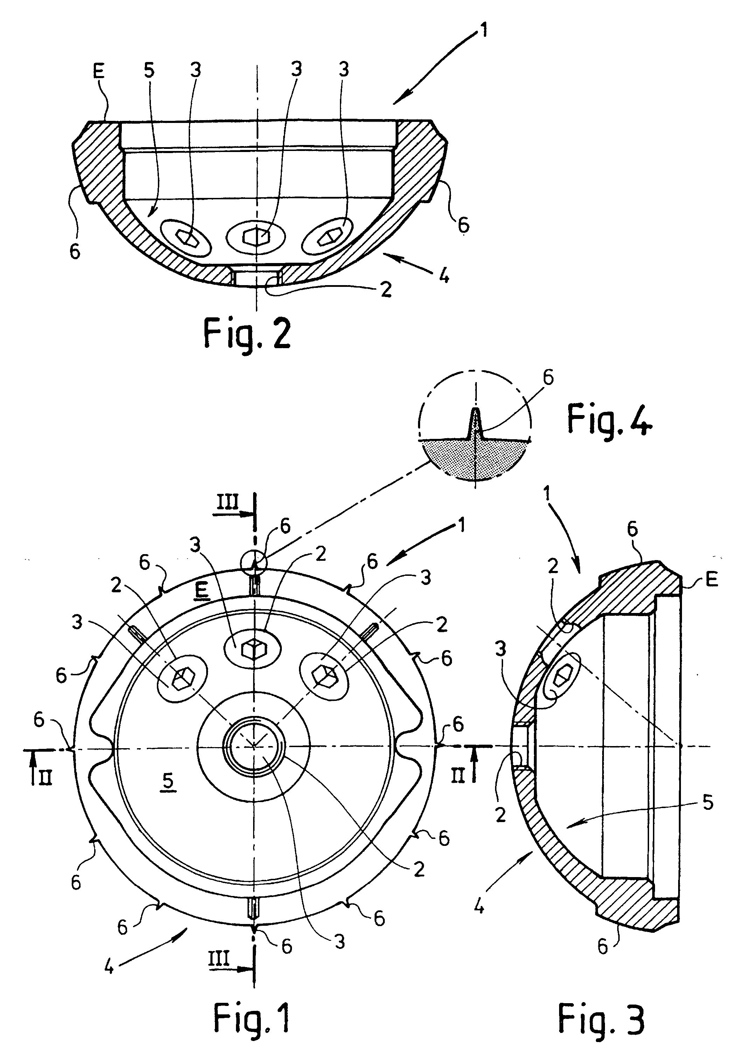

La figure 1 est une vue en plan intérieur, de la cupule cotyloïdienne selon l'invention.
La figure 2 est une vue en coupe transversale selon la ligne II/II de la figure 1.
La figure 3 est une vue en coupe transversale selon la ligne III/III de la figure 1.
La figure 4 est une vue de détail de la figure 1.

La cupule cotyloïdienne 1 désignée globalement sur les figures est destinée à recevoir une tête sphérique d'une tige fémorale ou humérale (non représentée) dans laquelle sont percés un certain nombre de trous filetés 2 initialement obstrués par des bouchons 3 et libérés à la demande pour être traversés par des vis de fixation (non représentées) dans le cotyle humain, avant mise en place d'un insert interne en matière plastique (non représenté).

Selon l'invention, la cupule reçoit sur sa face externe 4 un revêtement de titane poreux destiné à assurer l'étanchéité de la cupule 1, notamment au niveau des trous filetés 2 non utilisés demeurant obstrués par leur bouchon respectif 3.

Préférentiellement, le revêtement de titane poreux répond à la norme ISO 5832-3 non allié, d'épaisseur comprise entre 5 et 150 microns.

Il s'agit en fait, d'un revêtement de titane T40 qui a donné des résultats particulièrement bon lors des essais.

Selon une autre caractéristique de l'invention le revêtement de titane poreux constitue une sous couche à un revêtement d'hydroxy-apatite projeté successivement sur celle-ci afin de favoriser la repousse osseuse.

Il s'agira d'un hydroxy-apatite qui a également donné d'excellent résultat lors des essais.

Le revêtement d'hydroxy-apatite a une épaisseur comprise entre 20 et 200 microns.

La composition chimique du revêtement d'hydroxy-apatite est : Ca5(Po4)3 OH>97%.

Les applications de revêtement de titane poreux, puis de revêtement d'hydroxy-apatite sont effectuées par projection à l'aide d'une torche à plasma atmosphérique.

Selon une autre caractéristique de l'invention, préalablement au dépôt de titane puis d'hydroxy-apatite sur la surface 4 externe de la cupule 2, les extrémités des bouchons d'obstruction 3 débouchant des trous filetés 2 ainsi que leurs têtes débouchant sur la surface interne 5 de ladite cupule 1, sont rectifiées simultanément aux dites surfaces interne et externe, de manière à ce que les extrémités des vis desdits bouchons 3 ainsi que leurs têtes présentent un état de surface se confondant avec lesdites surfaces interne 5 et externe 4 de la cupule 2, permettant de parfaire l'étanchéité au niveau des extrémités des bouchons 3 et d'éviter le fluage du matériau plastique constituant l'insert interne.

En fait, les ébauches de bouchons sont arasées une fois vissées dans la cupule 1 par tournage des demi-sphères intérieurement et extérieurement.

Selon une autre caractéristique de l'invention la cupule cotyloïdienne est munie sur sa surface extérieure hémisphérique (4) d'une pluralité d'ailettes tranchantes (6), formant des portions de méridiens réparties symétriquement dans une zone périphérique située à proximité de sa partie équatoriale E, de manière à assurer la stabilité primaire en rotation et en bascule de ladite cupule (1) .

La stabilité primaire en rotation et en bascule est assurée en fait par douze ailettes tranchantes situées à la partie équatoriale E.

D'autre relief comme des pointes divergentes, ou des tétons s'opposent toujours à une impaction à force.

Seules des ailettes positionnées dans le sens axial de compression peuvent être efficaces dans le cas d'une cupule implantée en pression ajustage.

Celles-ci auront toujours un effet directionnel favorable sur le positionnement à long terme.

Si la stabilisation primaire n'est pas satisfaisante, les trois trous 2 permettent une utilisation de vis de fixation complémentaire (non représentées).

L'ancrage osseux se produit sur la surface de contact externe de la cupule et sur les ailettes. Le revêtement d'hydroxy-apatite a pour but d'accélérer cette repousse osseuse. Les ailettes sont à l'origine d'une ostéoformation intense. Cette repousse osseuse a été caractérisée par des études histologiques qui sont de notoriété publique.

La cupule a été conçue avec une épaisseur minimum de métal de 3mm afin de préserver une épaisseur maximum de l'insert en polyéthylène. Le mouvement entre l'insert et la cupule a été minimisé du fait que ceux-ci sont en contact. Egalement le nombre de trous 3 destinés aux vis est limité afin de réduire à long terme le fluage de l'insert.

## Revendications

1. Cupule cotyloïdienne destinée à recevoir une tête sphérique d'une tige fémorale ou humérale, dans laquelle sont percés un certain nombre de trous filetés (2) initialement obstrués par des bouchons (3) et par des vis de fixation dans le cotyle humain avant mise en place d'un insert interne en matière plastique, **caractérisée en ce qu'**elle reçoit sur sa face externe (4) un revêtement de titane poreux destiné à assurer l'étanchéité de la cupule notamment au niveau des trous (2) non utilisés, demeurant obstrués par leurs bouchons respectifs (3), lequel revêtement de titane poreux constitue une sous couche à un revêtement d'hydroxy-apatite projeté successivement sur celle-ci afin de favoriser la repousse osseuse.

2. Cupule cotyloïdienne selon la revendication 1, **caractérisée en ce que** le revêtement de titane poreux répond à la norme ISO 5832-3 non allié, d'épaisseur comprise entre 5 et 150 microns.

3. Cupule cotyloïdienne selon l'une des revendications 1 ou 2, **caractérisée en ce que** le revêtement d'hydroxy-apatite à une épaisseur comprise entre 20 et 200 microns.

4. Cupule cotyloïdienne selon l'une des revendications 1 à 3, **caractérisée en ce que** la composition chimique du revêtement d'hydroxy-apatite est : Ca5(Po4)3 OH>97%

5. Cupule cotyloïdienne selon l'une des revendications 1 à 4, **caractérisée en ce qu'**elle est munie sur sa surface extérieure hémisphérique (4) d'une pluralité d'ailettes tranchantes (6), formant des portions de méridiens réparties symétriquement dans une zone périphérique située à proximité de sa partie équatoriale E, de manière à assurer la stabilité primaire en rotation et en bascule de ladite cupule (1).

6. Procédé de fabrication d'une cupule cotyloïdienne selon l'une des revendications 1 à 5, **caractérisée en ce que** les applications de revêtement de titane poreux, puis de revêtement d'hydroxy-apatite sont effectuées par projection à l'aide d'une torche à plasma atmosphérique.

7. Procédé de fabrication d'une cupule cotyloïdienne selon la revendication 6, **caractérisée en ce que** préalablement au dépôt de titane puis d'hydroxy-apatite sur la surface (4) externe de la cupule (2), les extrémités des bouchons d'obstruction (3) débouchant des trous filetés (2) ainsi que leurs têtes débouchant sur la surface interne (5) de ladite cupule (1), sont rectifiées simultanément aux dites surfaces internes et externe, de manière à ce que les extrémités des vis desdits bouchons (3) ainsi que leurs têtes présentent un état de surface se confondant avec lesdites surfaces interne (5) et externe (4) de la cupule (2), permettant de parfaire l'étanchéité au niveau des extrémités des bouchons (3) et d'éviter le fluage du matériau plastique constituant l'insert interne.

## Claims

1. Acetabular cup intended to receive a spherical head of a femoral or humeral shaft, in which cup a number of threaded holes (2) are formed which are initially occluded by plugs (3) and by screws for fixation in the human acetabulum before placement of an internal plastic insert, **characterized in that** it receives, on its outer face (4), a porous titanium covering intended to ensure leaktightness of the cup in particular in the area of the holes (2) not used and remaining occluded by their respective plugs (3), which porous titanium covering constitutes an underlayer for a hydroxyapatite covering sprayed successively onto it in order to promote bone growth.

2. Acetabular cup according to Claim 1, **characterized in that** the porous titanium covering complies with ISO 5832-3, unalloyed, with a thickness of between 5 and 150 microns.

3. Acetabular cup according to either of Claims 1 and 2, **characterized in that** the hydroxyapatite covering has a thickness of between 20 and 200 microns.

4. Acetabular cup according to one of Claims 1 to 3, **characterized in that** the chemical composition of the hydroxyapatite covering is: Ca5(PO4)3 OH>97%.

5. Acetabular cup according to one of Claims 1 to 4, **characterized in that** it is provided, on its outer hemispherical surface (4), with a plurality of cutting fins (6) forcing meridian portions distributed symmetrically in a peripheral zone situated in proximity to its equatorial part E, in such a way as to ensure primary stability, in rotation and in tilting, of said cup (1).

6. Method for producing an acetabular cup according to one of Claims 1 to 5, **characterized in that** the porous titanium covering, then the hydroxyapatite covering, are sprayed on using an atmospheric plasma torch.

7. Method for producing an acetabular cup according to Claim 6, **characterized in that**, prior to the deposition of titanium then of hydroxyapatite on the outer surface (4) of the cup (1), the ends of the occlusion plugs (3) emerging from the threaded holes (2) and their heads emerging from the inner surface (5) of said cup (1) are corrected simultaneously to said inner and outer surfaces, in such a way that the ends of the screws of said plugs (3) and their heads have a surface state coincident with said inner (5) and outer (4) surfaces of the cup (1), making it possible tc perfect the leaktightness at the ends of the plugs (3) and to avoid creep of the plastic constituting the internal insert.

## Patentansprüche

1. Cupula für eine Gelenkpfanne, die für das Aufnehmen eines Kugelkopfs eines femoralen oder humeralen Schafts vorgesehen ist, der mit einer bestimmten Anzahl von Gewindelöchem (2) durchsetzt ist, welche anfänglich durch Verschlußkappen (3) und durch in der menschlichen Kondyle vorgesehene Fixationsschrauben verschlossen sind, bevor ein innerer Einsatz aus Kunststoffmaterial eingesetzt wird, **dadurch gekennzeichnet, daß** sie an ihrer Außenseite (4) eine poröse Titanschicht aufnimmt, um die Abdichtung der Cupula insbesondere in der Höhe der nicht verwendeten Löcher (2) zu gewährleisten, welche durch ihre jeweiligen Verschlußkappen (3) verschlossen bleiben, wobei die poröse Titanschicht eine Unterschicht einer Hydroxylapatit-Schicht darstellt, die danach auf dieser aufgebracht ist, um das Nachwachsen des Knochens zu fördern.

2. Cupula für eine Gelenkpfanne nach Anspruch 1, **dadurch gekennzeichnet, daß** die nicht verbundene, poröse Titanschicht, deren Stärke zwischen 5 und 150 µm beträgt, die Norm ISO 5832-3 erfüllt.

3. Cupula für eine Gelenkpfanne nach Anspruch 1 oder 2, **dadurch gekennzeichnet, daß** die Hydroxylapatit-Schicht eine Stärke zwischen 20 und 200 µm aufweist.

4. Cupula für eine Gelenkpfanne nach einem der Ansprüche 1 bis 3, **dadurch gekenn-zeichnet, daß** die chemische Zusammensetzung der Hydroxylapatit-Schicht wie folgt ist:
Ca₅ (PO₄) ₃ OH > 97% .

5. Cupula für eine Gelenkpfanne nach einem der Ansprüche 1 bis 4, **dadurch gekennzeichnet, daß** auf ihrer äußeren Hemisphär-Oberfläche (4) eine Vielzahl von Schneidrippen (6) vorgesehen sind, die Meridianabschnitte bilden, welche symmetrisch in einem äußeren Bereich in der Nähe ihres äquatorialen Abschnitts E in einer Weise angeordnet sind, die die primäre Stabilität beim Drehen und Kippen der Cupula gewährleistet.

6. Herstellungsverfahren einer Cupula für eine Gelenkpfanne nach einem der Ansprüche 1 bis 5, **dadurch gekennzeichnet, daß** das Aufbringen der porösen Titanschicht und danach der Hydroxylapatit-Schicht durch Aufbringen mit Hilfe eines atmosphärischen Plasmabrenners durchgeführt wird.

7. Herstellungsverfahren einer Cupula für eine Gelenkpfanne nach Anspruch 6, **dadurch gekennzeichnet, daß** vor dem Aufbringen des Titans und danach der Hydroxylapatitschicht auf die Außenfläche (4) der Kapsel (2) die Außenteile der Verschlußkappen (3), welche in die Gewindelöcher (2) einmünden, sowie deren in der Innenfläche (5) der Kapsel (1) mündenden Köpfe gleichzeitig mit der Innen- und Außenfläche in einer Weise ausgerichtet werden, daß die Oberflächen der Außenteile der Gewinde der Kappen (3) und ihre Köpfe mit der Innen- (5) und der Außenfläche (4) der Kapsel (2) zusammenfallen, wodurch die vollständige Abdichtung auf der Höhe der Außenteile der Kappen (3) gewährleistet ist und das Kriechen von Kunststoffmaterial, das den inneren Einsatz bildet, verhindert wird.
